# FASCICULE DE BREVET EUROPEEN

(11) **EP 1 131 056 B1**
(45) Date de publication et mention de la délivrance du brevet: **19.10.2005**
(21) Numéro de dépôt: 99956102.0
(22) Date de dépôt: 19.11.1999
(51) Int. Cl.: A61K 9/16, A61K 9/51

(54) **PARTICULES A BASE DE POLYAMINOACIDE(S) ET LEURS PROCEDES DE FABRICATION**
PARTIKEL AUF POLYAMINOSÄURE(N)BASIS SOWIE DEREN HERSTELLUNGSVERFAHREN
PARTICLES BASED ON POLYAMINO-ACID(S) AND METHODS FOR PREPARING SAME

(30) Priorité: 20.11.1998 FR 9814863
(43) Date de publication de la demande: 12.09.2001
(73) Titulaire: FLAMEL TECHNOLOGIES, 69693 Venissieux Cédex (FR)
(72) Inventeur: HUILLE, Sylvain, F-69003 Lyon (FR); NICOLAS, Florence, 69740 Genas (FR); BRYSON, Nathan, F-69390 Millery (FR); SOULA, Gérard, F-69330 Meyzieu (FR)
(74) Mandataire: Fleurance, Raphael
(86) Numéro de dépôt international: PCT/FR1999/002859
(87) Numéro de publication internationale: WO 2000/030618

(56) Documents cités:
- EP-A- 0 734 720
- TSCHUTSUMIUCHI K. ET AL: 'Synthesis of Polyoxazolidine...' MACROMOLECULES vol. 30, 1997, pages 4013 - 4017, XP000692821

## Description

Le domaine de la présente invention est celui des Particules de Vectorisation (**PV**), utiles pour l'administration de principes actifs (**PA**). Ces derniers sont, de préférence, des médicaments ou des nutriments pour l'administration à un organisme animal ou humain par voie orale, nasale, vaginale, oculaire, sous-cutanée, intraveineuse, intramusculaire, intradermique, intrapéritonéale, intracérébrale, parentérale, etc.. Mais il peut s'agir aussi de produits phytosanitaires, tels que des herbicides, des pesticides, des insecticides, des fongicides, etc. En terme de nature chimique, les **PA** plus particulièrement, mais non limitativement, concernés par l'invention sont, par exemple, des protéines, des glycoprotéines, des peptides, des polysaccharides, des lipopolysaccharides, des oligonucléotides et des polynucléotides.

La présente invention concerne, plus précisément, des Particules de Vectorisation, avantageusement de type submicronique, à base de polyaminoacides (PAA). La présente invention vise aussi bien les particules nues en tant que telles, que les systèmes de vecteurs de **PA**, constitués par les particules chargées .par le (ou les) **PA** considéré(s). La présente invention a également trait à des suspensions colloïdales aqueuses comprenant ces **PV.**

L'invention concerne, également, des procédés de préparation desdites particules et suspensions colloïdales, avec et sans **PA**.

L'encapsulation de **PA** dans des **PV** a notamment, pour but d'augmenter la biodisponibilité desdits **PA**. De nombreuses techniques d'encapsulation ont déjà été proposées. De telles techniques visent, d'une part, à permettre le transport du **PA** jusqu'à son site d'action thérapeutique, tout en le protégeant contre les agressions de l'organisme (hydrolyse, digestion enzymatique, etc.) et, d'autre part, à contrôler la libération du **PA** sur son site d'action, afin de maintenir la quantité disponible pour l'organisme au niveau désiré. Les **PA** concernés par ces avatars de transport et de séjour dans l'organisme sont, par exemple, des protéines mais peuvent être, également, des produits tout autres, d'origine synthétique ou naturelle. La revue de M. J. HUMPHREY (Delivery System for peptide Drugs, éditée par S. DAVIS et L. ILLUM, Plenum Press, N. Y., 1986), fait état de la problématique concernant l'amélioration de la biodisponibilité des **PA** et l'intérêt des systèmes de vectorisation et de libération contrôlée.

Parmi tous les matériaux envisageables pour former des **PV**, les polymères sont de plus en plus utilisés, du fait de leurs propriétés intrinsèques. S'agissant du cahier des charges que l'on souhaite obtenir pour les **PV**, il est particulièrement exigeant et comprend, notamment, les spécifications suivantes.
**1** - Il devrait, avantageusement, être possible d'obtenir une répartition granulométrique contrôlée, et adaptée au mode d'administration choisi et/ou au site thérapeutique visé.
**2** - Il est souhaitable que les **PV** assurent la protection du **PA** jusqu'au site de libération.
**3** - Les PV devraient, avantageusement, contrôler la vitesse de libération du **PA.**
**4** - Il est préférable que le polymère, constituant les **PV,** soit biocompatible, éliminable (par excrétion), et/ou biodégradable et, encore mieux, qu'il soit métabolisé en produits non toxiques pour l'organisme.
**5** - Il est également avantageux que le polymère, constitutif des **PV**, n'induise pas de réponse immunitaire.
**6** - Enfin, il est également souhaitable que les **PV** et les systèmes **PV-PA** puissent être obtenus par un procédé non dénaturant pour le **PA**.

Les propositions techniques antérieures, décrites infra, ont tenté de satisfaire à l'ensemble de ces spécifications. A titre d'illustration, on citera les propositions antérieures **(a)** à **(h)** : selon une première approche, qui comprend les propositions **(a)** à **(d)**, l'inclusion du principe actif s'opère lors de la formation des supports de vectorisation; selon une seconde approche, citées dans les propositions **(e)** à **(h)**, on produit des **PV** aptes, une fois fabriquées, à s'associer spontanément par absorption au **PA**.
**(a)** Le brevet US-A-5 286 495 concerne un procédé d'encapsulation par vaporisation de protéines en phase aqueuse, à l'aide de matériaux ayant des charges opposées, à savoir : l'alginate (chargé négativement) et la polylysine (chargée positivement). Ce procédé de fabrication permet de produire des particules de taille supérieure à 35 µm.
**(b)** Par ailleurs, les techniques d'émulsion sont couramment utilisées pour préparer des microparticules chargées de **PA**. Par exemple, les demandes de brevets WO 91/06286, WO 91/06287 et WO 89/08449 divulguent de telles techniques d'émulsion dans lesquelles on a recours à des solvants organiques pour solubiliser des polymères, par exemple de type polylactique. Mais il s'est avéré que les solvants peuvent être dénaturants, notamment pour les **PA** peptidiques ou polypeptidiques.
**(c)** On connaît, également, des **PV** biocompatibles, formées en solution aqueuse et appelées protéinoïdes, décrites dès 1970 par W. FOX et K. DOSE dans "Molecular Evolution and the origin of Life", Ed. Marcel DEKKER Inc. (1977). Ainsi, la demande de brevet WO 88/01 213 propose un système à base d'un mélange de polypeptides artificiels obtenus par condensation thermique d'acides aminés. Les microparticules selon cette invention sont obtenues par un changement de pH qui provoque la précipitation des particules protéinoïdes.
**(d)** On mentionnera également, pour mémoire, les brevets US 4 351 337 et 4 450 150 qui relèvent d'un domaine différent de celui de la vectorisation de **PA** propre à l'invention. Ces brevets divulguent des implants massiques fixés et localisés à des endroits bien précis de l'organisme. Les implants sont réalisés à partir de matériaux polymères du genre poly-α-aminoacide (Leu/GluOH et GluOEt/GluOH respectivement). Selon l'enseignement de ce brevet, les polyaminoacides préférés sont ceux riches en aminoacide hydrophobe (e. g. plus de 50 % de Leucine ou de Glu OEt) et insolubles dans l'eau. Le **PA** peut être incorporé dans une solution de poly-α-aminoacide dans un solvant organique. Cette solution est celle utilisée pour former l'implant par moulage/séchage (évaporation). Selon une autre variante, le **PA** peut être compris dans le noyau d'une microcapsule dont l'enveloppe est obtenue à partir d'une solution de copolymère et dont le diamètre est supérieur ou égal à 5 000 µm. Le noyau peut être constitué de **PA** pur ou d'une matrice de copolymère incluant le **PA** et obtenue à partir de la solution de PAA dans un solvant organique.
**(e)** La demande de brevet PCT WO/FR 97/02 810 divulgue une composition pour la libération contrôlée de principes actifs, comprenant une pluralité de particules lamellaires d'un polymère biodégradable, au moins en partie cristallin (polymère d'acide lactique) et d'un principe actif absorbé sur lesdites particules. La libération du principe actif s'opère par désorption.
**(f)** La publication "*CHEMISTRY LETTERS 1995, 707, AKIYOSHI et al"* concerne la stabilisation d'insuline par complexation supramoléculaire avec des nanoparticules formées d'une dizaines de chaînes de polysaccharide rendues hydrophobes par greffage de cholestérol.
**(g)** L'article paru dans *"MACROMOLECULES 1997, 30, 4013-4017"* décrit des copolymères composés d'un bloc peptidique à base de L-phénylalanine, de γ-benzyl-L-glutamate ou de O-(tétra-O-acétyle-β-D-glucopyranosyl)-L-sérine, et un bloc synthétique, tels que la poly(2-méthyle-2-oxazoline) ou la poly(2-phényle-2-oxazoline). Certains des ces polymères s'agrègent en milieu aqueux pour former des particules de 400 nm, capables de s'associer avec une enzyme, la lipase.
**(h)** Le brevet FR 95-03978 a pour objet des particules de polyaminoacides, utiles pour la vectorisation de principes actifs et caractérisées en ce que leurs polyaminoacides constitutifs comprennent au moins deux types d'aminoacides récurrents AAN (neutres, hydrophobes) et AAI (ionisables et hydrophiles). Les particules sont obtenues spontanément par dispersion de la poudre de polyaminoacide dans une solution aqueuse. Les particules ainsi obtenues s'associent spontanément en suspension aqueuse avec des principes actifs, par exemple de nature protéique.
   Ces propositions techniques antérieures satisfont peu ou prou aux spécifications du cahier des charges indiqué supra. Néanmoins, de telles particules de vectorisation de principes actifs restent perfectibles.
   Dans cet état de fait, l'un des objectifs essentiels de la présente invention est de perfectionner la proposition technique antérieure référencée **(h)** FR 95-03 978, en fournissant de nouvelles **PV** à base de poly-α-aminoacides (PAA) linéaires, amphiphiles et dont les caractères hydrophile et hydrophobe sont, respectivement, apportés par les acides aminés de la chaîne principale du polymère et par des radicaux hydrophobes attachés latéralement à une fraction des aminoacides par une liaison covalente. Ce perfectionnement concerne plus précisément un moyen de maîtriser les caractéristiques de l'association entre **PA** et **PV** afin d'améliorer le taux de chargement en **PA**, et/ou d'améliorer la cinétique de libération du **PA**.
   Un autre objectif essentiel de l'invention est de fournir une suspension colloïdale aqueuse de particules de vectorisation de principes actifs, qui comprenne des particules de PAA satisfaisant aux spécifications visées ci-dessus et qui constitue une forme galénique appropriée et convenable pour une administration, par exemple orale, à l'homme ou l'animal.
   Un autre objectif essentiel de l'invention est de proposer un procédé de préparation de particules de PAA utiles, notamment, comme vecteurs de principes actifs, ledit procédé se devant d'être encore plus économique, plus simple à mettre en oeuvre, non dénaturant pour les principes actifs et devant en outre toujours permettre une maîtrise fine de la granulométrie moyenne des particules obtenues.
   Un autre objectif essentiel de l'invention est l'utilisation des susdites suspensions et particules pour la préparation de médicaments (e. g. vaccins) et/ou de nutriments, en particulier pour administration notamment orale, nasale, vaginale, oculaire, sous-cutanée, intraveineuse, intramusculaire, intradermique, intrapéritonéale, intracérébrale ou parentérale, de principes actifs, tels que des protéines, des glycoprotéines, des peptides, des polysaccharides, des lipopolysaccharides, des oligonucléotides et des polynucléotides.
   Un autre objectif essentiel de la présente invention est de fournir des suspensions de **PV**, en partie submicroniques et microniques, à base de PAA et susceptibles de servir de vecteurs d'un **PA**, en particulier médicamenteux et/ou nutritionnel pour l'administration dudit **PA** à un organisme humain ou animal.
   Un autre objectif de la présente invention est de fournir un médicament, du type système à libération prolongée de principes actifs, qui soit aisé et économique à produire et qui soit, en outre, biocompatible et apte à assurer un très haut niveau de biodisponibilité du **PA**.
   Un autre objectif essentiel de l'invention est de fournir un système de vectorisation de vaccin, qui soit non immunogène intrinsèquement et en combinaison avec un ou plusieurs antigènes.
   Les objectifs relatifs aux produits, parmi d'autres, sont atteints par la présente invention qui concerne, tout d'abord, des particules susceptibles d'être utilisées, notamment pour la vectorisation (**PV**) de principe(s) actif(s), du type de celles :
   - à base de polyaminoacides (PAA) amphiphiles, linéaires, à enchaînements α-peptidiques ;
   - aptes à se former spontanément par mise en contact des PAA avec un milieu liquide, de préférence avec de l'eau, dans lequel la partie hydrophile des PAA se solubilise plus que la partie hydrophobe desdits PAA, de manière que ces derniers précipitent en s'organisant en arrangements supra-moléculaires discrets ;
   - de taille moyenne inférieure à 200 µm, de préférence à 100 µm et, plus préférentiellement encore, comprise entre 0,01 et 20 µm ;
   - et aptes à s'associer avec au moins un **PA** et à relarguer celui-ci in vivo, de manière prolongée et contrôlée ;
   caractérisées :
   - en ce que les aminoacides récurrents (AAr) constitutifs de la chaîne principale des PAA sont identiques ou différents entre eux et sont choisis parmi les aminoacides de type acide et de préférence dans le groupe comprenant l'acide glutamique et/ou l'acide aspartique et/ou leurs sels, respectivement les glutamates et les aspartates,
   - et en ce que certains de ces AAr sont porteurs chacun d'au moins un groupement R⁰ hydrophobe, ces groupements hydrophobes R⁰ étant identiques ou différents entre eux.

L'une des conditions, pour que les particules selon l'invention se forment, est que la partie hydrophile des PAA se solubilise plus dans le milieu liquide de suspension que leur partie hydrophobe.

Les aminoacides récurrents (AAr) sélectionnés conformément à l'invention, sont les aminoacides à fonctions carboxyliques (Glu et Asp) sous forme COOH ou sous forme de sels (carboxylates) COO⁻, X⁺ ; avec X correspondant aux métaux alcalins, de préférence à Na.

Il est du mérite de la demanderesse d'avoir choisi, à titre de matériau constitutif des **PV**, une classe particulière de poly-α-aminoacides dont les (co)monomères sont des aminoacides récurrents (AAr) de nature polaire et qui ont été rendus amphiphiles en modifiant le caractère hydrophile par l'apport des chaînes latérales hydrophobes sur une fractions des AAr. Cette amphiphilie acquise confère aux PAA la possibilité de former des suspensions colloïdales de **PV**, compatible avec le pH des milieux physiologiques rencontrés dans les applications visées.
Cette sélection permet, avantageusement, de disposer d'un plus grand choix quant à la nature des groupements hydrophobes, et ainsi d'un meilleur moyen de contrôle de l'hydrophobie du polymère et du **PV**, ce qui permet d'optimiser l'association et la libération des **PA**.

La structure du polymère PAA, la nature des acides aminés Asp et Glu et les radicaux hydrophobes sont choisis de telle façon que les chaînes de polymères se structurent spontanément sous forme de particules de petite taille, stables en milieu physiologique.

La structure du polymère PAA, la nature des acides aminés Asp et Glu et les radicaux hydrophobes sont choisis de telle façon que les **PV** encapsulent des protéines, ou autres **PA**, en milieu aqueux par un mécanisme spontané et non dénaturant pour les protéines.

La structure du polymère PAA, la nature des acides aminés Asp et Glu et les radicaux hydrophobes sont choisis de telle façon que les **PV** libèrent les **PA** en milieu physiologique et, plus précisément, in vivo, la cinétique de libération étant une fonction de la nature du polymère et des **PV** qu'il est susceptible de former.

Sans que cela ne soit limitatif, cette sélection vise, plus spécifiquement, des PAA dont la chaîne principale se compose d'AAr identiques entre eux. La structure primaire des PAA peut être du type ordonné, séquentiel alterné (PAA blocs) ou du type désordonné, séquentiel aléatoire (PAA statistique). Selon une caractéristique préférée de l'invention, les PAA sont des polymères contenant jusqu'à environ 1 000 AAr et, de préférence, jusqu'à environ 500 AAr et, plus préférentiellement encore, jusqu'à environ 200 AAr.

Les groupements hydrophobes R⁰ participent à l'agrégation des chaînes polymères, qui est au coeur de la formation des **PV**. Suivant une disposition préférée de l'invention, ces groupements R⁰ sont identiques ou différents entre eux et sont sélectionnés dans le groupe comprenant :
**(i)** les alkyles, les acyles ou les alcényles linéaires ou ramifiés, de préférence linéaires en C₁-C₂₀ et, plus préférentiellement encore, en C₂-C₁₈ ;
**(ii)** les groupements hydrocarbonés contenant un ou plusieurs hétéroatomes, de préférence ceux contenant de l'oxygène et/ou du soufre et, plus préférentiellement encore, ceux de formule suivante : 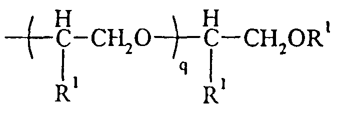 dans laquelle :
   - les radicaux R¹ sont identiques ou différents entre eux et correspondent à l'hydrogène ou à un radical répondant à la même définition que celle donnée supra au point (i),
   - q = 1 à 100 ;
      **(iii)** les aryles, les aralkyles ou les alkylaryles, de préférence les aryles ;
      **(iv)** les dérivés naturels hydrophobes, de préférence le cholestérol, les phosphatidylcholines et les diacylglycérols.
Par "groupements hydrocarbonés", on entend au sens de la présente invention, des groupements comprenant notamment des atomes d'hydrogène et de carbone.

De préférence, les groupements R⁰ sont sélectionnés dans le groupe de radicaux suivants : méthyle, éthyle, propyle, docédyle, hexadécyle, octadécyle.

Conformément à l'invention, chaque groupement hydrophobe R⁰ est attaché à la chaîne principale par une liaison covalente clivable, de préférence par hydrolyse chimique et/ou enzymatique, ladite liaison étant plus spécialement de type ester ou amide.

Avantageusement, la fraction de monomères AAr porteurs de groupements hydrophobes représente une proportion supérieure ou égale à 3%, de préférence comprise entre 3-70 % et, plus préférentiellement encore, entre 13 et 60 %.

En pratique, les PAA constitutifs des **PV** de la suspension selon l'invention sont, par exemple:
* des polymères à base de glutamate :
   - poly(glutamate de sodium)-bloc-(glutamate de méthyle),
   - poly(glutamate de sodium)-bloc-(glutamate d'éthyle),
   - poly(glutamate de sodium)-bloc-(glutamate de propyle),
   - poly(glutamate de sodium)-bloc-(glutamate de octadécyl),
   - poly(glutamate de sodium)-bloc-(glutamate de benzyle),
   - poly(glutamate de sodium)-co-(glutamate de méthyle),
   - poly(glutamate de sodium)-co-(glutamate d'éthyle),
   - poly(glutamate de sodium)-co-(glutamate de propyle),
   - poly(glutamate de sodium)-co-(glutamate de dodécyle),
   - poly(glutamate de sodium)-bloc-(glutamate de octadécyle),
* des polymères à base d'aspartate :
   - poly(aspartate de sodium)-bloc-(aspartate de méthyle),
   - poly(aspartate de sodium)-bloc-(aspartate d'éthyle),
   - poly(aspartate de sodium)-bloc-(aspartate de propyle),
   - poly(aspartate de sodium)-bloc-(aspartate d'octadécyl),
   - poly(aspartate de sodium)-bloc-(aspartate de benzyle).

Avantageusement, les particules **PV** de taille moyenne comprise entre 0,01 µm et 0,5 µm, de préférence entre 0,01 et 0,2 µm. Au sens de l'invention, on entend, par taille ou granulométrie moyenne, la moyenne arithmétique des diamètres en volume [D4,3] établis par diffraction laser et le diamètre de giration mesuré par diffusion élastique de la lumière.

L'un des atouts de l'invention est d'être parvenu à un très bon contrôle de la granulométrie moyenne des ces entités matricielles et de leur répartition granulométrique. Ce contrôle passe par l'atteinte de tailles de particules extrêmement réduites, de l'ordre de quelques nanomètres et de très faible polydispersité, sachant qu'il est possible d'augmenter la taille de ces nanoparticules par agrégation.

Le contrôle de la taille **PV** s'opère par l'intermédiaire de la composition en AAr et R⁰ des polyaminoacides mais aussi, pour une même composition, par le choix d'une structure bloc et du procédé d'obtention.

Selon une alternative quant à la taille des **PV** de la suspension selon l'invention, lesdites particules de vectorisation **PV** sont agrégées afin d'obtenir de nouvelles particules de taille supérieure.
Le matériau PAA constitutif des **PV** selon l'invention est, de préférence, un homopolymère ou un copolymère dont l'AAr = Asp et/ou Glu et qui a été rendu amphiphile en modifiant le caractère hydrophile de cet AAr par apport de chaînes latérales hydrophobes R⁰ sur une fraction des AAr. Cette amphiphilie acquise permet de réunir au moins trois propriétés nouvelles et surprenantes :
**1.** la première est la possibilité de former spontanément des suspensions colloïdales de **PV** compatibles avec le pH des milieux physiologiques rencontrés dans les applications thérapeutiques visées ;
**2.** la deuxième est l'association spontanée des **PV** avec des **PA** en l'absence d'autre agent que l'eau qui leur sert de solvant, qui, dans le cas des protéines, n'est pas dénaturant ;
3. la troisième est la possibilité de libérer le **PA** du complexe d'association **PA-PV**, dans les conditions physiologiques, avec des profils pharmacocinétique et pharmacodynamique, qui permettent d'envisager des utilisations dans le domaine thérapeutique.

Les **PA**, en particulier de nature protéique, sont, en fait, piégés par les particules de vectorisation **PV** qui peuvent être assimilées à des matrices formées d'eau et de PAA au sein desquelles se sont dispersés le ou les **PA**. Leur libération s'opère, soit par dissociation spontanée, soit au fur et à mesure de la dégradation des PAA et de la déstructuration subséquente des particules. Le piégeage des **PA** est réalisé spontanément par simple mélange de ceux-ci dans une suspension aqueuse colloïdale de **PV** selon l'invention.

Les particules **PV** chargées par au moins un principe actif **PA** constituent un autre objet de la présente invention.

Selon un autre de ses aspects, en amont des particules, qui forment son objet essentiel, l'invention concerne, en outre, les précurseurs des **PV** qui sont les PAA sélectionnés, tels que définis supra.

En aval des **PV**, l'invention englobe également la suspension colloïdale, de préférence aqueuse, de particules telles que définies supra.

Cette suspension colloïdale de **PV** chargées en **PA** peut être utilisée pour la vectorisation et le relargage prolongé et contrôlé dudit **PA** in vivo, à des fins thérapeutiques.

L'invention a trait, par ailleurs, à un procédé de préparation de la susdite suspension colloïdale de particules e. g. de vectorisation (**PV**) de principes actifs (**PA**),
caractérisé en ce qu'il consiste, essentiellement :
**I -** à mettre en oeuvre des PAA linéaires et à enchaînements α-peptidiques d'aminoacides récurrents (AAr) sélectionnés parmi les aminoacides naturels polaires que sont l'acide glutamique et l'acide aspartique et/ou parmi leurs sels, respectivement les glutamates et les aspartates ; certains de ces AAr étant porteurs chacun d'au moins un groupement hydrophobe R⁰, ces groupements R⁰ étant identiques ou différents entre eux ;
**II** - à placer les PAA amphiphiles dans un milieu solvant, de préférence aqueux;
**III** - puis à rendre insolubles au moins les AAr porteurs de R⁰ sans le milieu, de manière à faire précipiter ces AAr et à former ainsi des arrangements supra moléculaire discrets.

Selon une variante avantageuse de ce procédé, on incorpore au moins un principe actif dans le milieu liquide contenant les particules **PV,** de manière à obtenir une suspension colloïdale de **PV** chargées en principe(s) actif(s).

De plus, pour augmenter la taille des particules, il est envisageable de prévoir, dans ce procédé de préparation de la suspension, au moins une étape supplémentaire d'agrégation des particules, à l'aide d'au moins un agent d'agrégation constitué, par un sel et/ou un acide et/ou une base et/ou un polymère éventuellement ionique (e. g. polylysine, polyéthylène imine, etc.). Pour plus de détails, on se référera à la demande de brevet FR 95-03 918.

Après avoir passé en revue les caractéristiques essentielles de l'invention en termes de particules de précurseurs PAA desdites particules de suspension colloïdale de ces particules de préparation de cette suspension, il convient, à présent, de développer un peu plus, d'une part, l'aspect obtention des PAA et des particules chargées ou non en **PA** et, d'autre part, l'aspect application des **PV** et des suspensions de ces **PV** dans des systèmes médicamenteux à libération prolongée et contrôlée de **PA**.

Les PAA sont obtenus de manière connue en soi. A cet égard, on se reportera, par exemple, à : "*Encyclopedia of Polymer Science and Engineering, volume 12, p. 786 ; John Wiley & Sons*". Dans le cadre de l'invention, on préfère recourir aux techniques de polymérisation faisant intervenir des anhydrides de N-carboxy-α-aminoacides dont la préparation est donnée, e. g. dans : "*Biopolymers,* *15**, 1869 (1976) ".* S'agissant des techniques de polymérisation de ces monomères N-carboxy-α-aminoacides (NCA), elles sont détaillées dans l'ouvrage de H. R. KRICHELDORF "*α-Aminoacid-N-Carboxy Anhydride and Related Heterocycles*" *Springer Verlag (1987).*

La nature de la distribution des groupement hydrophobes sur les chaînes de polymères est statistique ou ordonnée, selon la voie de synthèse choisie. A cet égard, il existe une multitude de schémas réactionnels conduisant aux PAA sélectionnés comme matière première pour l'obtention des **PV** selon l'invention. Les trois schémas réactionnels sont donnés ci-après, à titre non limitatif :
**(i)** synthèse ou mise en oeuvre (étape **A**) d'un copolymère PAA comportant au moins deux types de groupements récurrents hydrophobes R⁰¹ et R⁰²,
   puis, par élimination sélective (étape **B**), au moins un d'entre eux est éliminé pour régénérer AAr dans son état non modifié (par exemple R⁰¹ = méthyle; R⁰² = benzyle ; l'étape **B** est une étape de débenzylation par hydrogénation, par addition du HBr, etc) ;
**(ii)** Synthèse ou mise en oeuvre (étape **A**) d'un homopolymère PAA hydrophobe comportant (exclusivement) des aminoacides récurrents hydrophobes (AAr-R⁰) qui sont tous porteurs d'un même groupement hydrophobe R⁰ (e. g. méthyle, éthyle, propyle, benzyle, stéaryle),
   puis l'élimination partielle (étape **B**) d'une partie des groupements R⁰ (e.g. de par hydrolyse ou saponification du PAA polyglutamate de méthyl) ;
**(iii)** synthèse ou mise en oeuvre (étape **A**) d'un homopolymère PAA hydrophile comportant (exclusivement) des aminoacides récurrents hydrophiles AAr,
   puis hydrophobisation partielle (étape **B**) par la formation d'une liaison covalente avec un ou plusieurs groupements R⁰ (e.g. par estérification à l'aide d'alcools gras ou par déplacement ionique à l'aide d'un halogénure d'alkyl).

Dans ces trois variantes **(i)**, **(ii)**, **(iii)**, la rotule de greffage des groupements hydrophobes R⁰ sur la chaîne latérale pendante des AAr, est avantageusement une fonction ester ou amide.

En pratique, les copolymères PAA hydrophobes mis en oeuvre dans la variante **(i)** sont, par exemple, des copolymères d'acide glutamique et/ou d'acide aspartique dont les fonctions carboxyliques latérales pendantes sont protégées par estérification selon des techniques connues de l'homme de l'art. Il peut s'agir, e. g., de copoly(stérylglutamate-benzylglutamate) obtenu par polymérisation des N-carboxyanhydrides des AAr₀₁ (Glu-O-stéaryl) et AAr₀₂ (Glu-O-benzyl).

L'hydrophilisation partielle du co-PAA résulte de l'élimination sélective de l'un des groupements R⁰, par hydrolyse et/ou saponification. Cela équivaut à une déprotection qui, dans l'exemple ci-dessus, est une débenzylation sélective. On exploite ici les différences de sensibilité au clivage des différents esters formant les groupements hydrophobes R⁰¹ et R⁰². A titre d'exemples de procédés de déprotection connus, on peut citer ceux par saponification des esters méthyliques (STAHMAN et coll. ; J. Biol. Chem., 197, 771 (1952) ; KYOWA HAKKO, FR 2 152 582) ou par débenzylation [BLOUT et coli. ; J. Amer. Chem. Soc., 80, 4631 (1958)].

Concernant la variante **(ii)**, l'homopolymère PAA hydrophobe est, par exemple, du polystéarylglutamate obtenu par polymérisation de NCA de Glu-O-stéaryl. L'hydrolyse partielle de cet homopolymère hydrophobe selon les méthodes de déprotection connues sus-visées conduit à un poly(glutamate)-co-(stéarylglutamate).

Dans la variante **(iii)**, l'homopolymère PAA hydrophile peut être, e. g., l'acide polyglutamique ou un polyglutamate, synthétisé par polymérisation de NCA d'acide glutamique. L'hydrophobisation de ce type de PAA poly Glu s'effectue, par exemple, par réaction avec du iodure de stéaryle en milieu basique. Cela conduit à du poly(glutamate)-co-(stéarylglutamate). Ce genre de techniques est notamment décrit dans [Polymer Bulletin, 32, 127 (1994)].

La formation des **PV** en milieu liquide, sous forme de suspension colloïdale conforme à l'invention, peut être réalisée pendant ou après la synthèse des PAA amphiphiles définis ci-dessus.

Selon une préférence de l'invention, la formation de particules s'opère par l'addition d'eau ou de sel à une solution de PAA, porteur de groupements R⁰, dissous dans un solvant. Cette opération consiste, préférentiellement, à diminuer la solubilité de la fraction hydrophobe pour qu'elle précipite et, ce faisant, entraîne la formation des particules. L'homme de l'art est en mesure de trouver d'autres moyens simples pour diminuer la solubilité de la partie hydrophobe du polymère, en modifiant, par exemple, la température, le solvant(s), ou en combinant des techniques différentes.

La préparation de la suspension colloïdale de **PV** est, avantageusement, suivie d'une étape de purification, impliquant les techniques connues à l'homme du métier, telles que la distillation, la filtration, la modification du pH, la chromatographie et/ou la dialyse. De telles méthodes permettent d'éliminer les sels ou les solvants indésirables. Après cette étape facultative de purification, on dispose d'une suspension colloïdale de **PV** que l'on peut utiliser directement, ou qu'il est envisageable d'isoler ou de recueillir, dans le cadre d'une étape **IV,** par tout moyen physique connu en soi et approprié, comme par exemple par filtration, par ultrafiltration, par séparation par gradient de densité, par centrifugation, par précipitation, éventuellement par ajout de sel, ou bien encore par lyophilisation.

Suivant une variante de l'invention, le procédé de préparation de la suspension colloïdale est caractérisé en ce que l'on incorpore au moins un principe actif dans le milieu liquide contenant les particules **PV**, de manière à obtenir une suspension colloïdale de **PV** chargées en ou associées avec un ou plusieurs principe(s) actif(s) **PA.**
Cette incorporation, qui conduit à un piégeage de **PA** par les **PV,** peut être réalisée de la manière suivante :
- mise en solution aqueuse de **PA**, puis ajout des **PV**, soit sous forme de suspension colloïdale, soit sous forme de **PV** isolées (lyophilisat ou précipitat) ;
- ou ajout de **PA**, soit en solution, soit à l'état pur ou préformulé, à une suspension colloïdale de particules **PV**, éventuellement préparée extemporanément par la dispersion de **PV** sèches dans un solvant approprié, tel que l'eau.

Le principe actif, susceptible d'être associé aux particules selon l'invention, peut être médicamenteux et/ou nutritionnel. Il est, de préférence, choisi parmi :
- les protéines et/ou les peptides parmi lesquels les plus préférentiellement retenus sont : les hémoglobines, les cytochromes, les albumines, les interférons, les antigènes, les anticorps, l'érythropoïétine, l'insuline, les hormones de croissance, le facteur IX, les interleukines ou leurs mélanges,
- les polysaccharides, l'héparine étant plus particulièrement sélectionnée,
- les acides nucléiques et, préférablement, les oligonucléotides d'ARN et/ou d'ADN,
- les vitamines, les acides aminés et les oligo-éléments.
- et leurs mélanges.

La présente invention vise, également, les précurseurs de ces particules **PV** constitués par les PAA particuliers définis ci-dessus et caractérisés en ce qu'ils contiennent des **PA** médicamenteux du genre de ceux énumérés ci-dessus en particulier l'insuline et/ou des **PA** formés par au moins un vaccin ou des **PA** nutritionnels, phytosanitaires ou cosmétiques.

La présente invention concerne également les suspensions colloïdales de **PV** caractérisées en ce qu'elles contiennent les mêmes **PA** que ceux énoncés ci-dessus, notamment pour les précurseurs de **PV**.

La présente invention concerne, enfin, les médicaments ou les spécialités pharmaceutiques ou nutritionnelles comprenant les **PV** chargées en **PA** et définies ci-dessus.
Selon un autre de ses aspects, l'invention vise également l'utilisation de ces **PV** chargées en **PA**, pour la fabrication de médicaments du type systèmes à libération contrôlée de **PA**.
Dans le cas de médicaments, il peut s'agir, par exemple, de ceux administrables, de préférence par voie orale, nasale, vaginale, oculaire, sous-cutanée, intraveineuse, intramusculaire, intradermique, intrapéritonéale, intracérébrale ou parentérale.

Les applications cosmétiques envisageables sont, par exemple, les compositions applicables par voies transdermiques.

Les produits phytosanitaires concernés peuvent être, par exemple, des herbicides, des pesticides, des insecticides, des fongicides, etc..

La présente invention a également pour objet les compositions phytosanitaires et cosmétiques comprenant des **PV** chargées en **PA** du type de ceux visés supra.

Les exemples qui suivent permettront de mieux comprendre l'invention dans ses différents aspects produit/procédé/application. Ces exemples illustrent la préparation de particules de polyaminoacides chargés ou non en principes actifs, de même qu'ils présentent les caractéristiques de structure et les propriétés de ces particules.

### EXEMPLES

### EXEMPLE 1 - PREPARATION DU POLYMERE - POLY(GLUTAMATE DE SODIUM)-BLOC-(GLUTAMATE DE METHYLE).

Les techniques utilisées, pour la polymérisation des **NCA** en polymères de structures en blocs ou statistiques sont connues de l'homme de l'art et sont détaillées dans l'ouvrage de H. R. KRICHELDORF "α-aminoacides-N-Carboxy Anhydrides and Related Heterocycles", Springer Verlag (1987). La synthèse suivante précise la synthèse de l'un d'entre eux.

*Synthèse de la poly(GluOMe)*_{*63*}*-poly(GluOBz)*_{*63*} *:* 10 g de NCA-GluOMe sont solubilisés dans un mélange de 150 ml de dioxane et 450 ml de toluène à 60 °C. 5 ml d'une solution de 0,91 g de benzylamine dans 50 ml de dioxane sont ajoutés au monomère en une fois. Après 1 h, on ajoute 14,1 g de NCA-GluOBz solubilisé préalablement dans un mélange de 20 ml dioxane et 60 ml de toluène. La polymérisation continue encore durant 19-24 h. Le polymère est précipité du milieu réactionnel dans 2 litres de méthanol, auxquels on ajoute encore 500 ml d'eau. Le solide obtenu est filtré, séché à l'étuve à 50 °C sous vide.
Rendement 90 %. Composition par RMN 1 h (TFA-d) 45 % molaire GluOBz. Viscosité réduite (0,5 % de TFA à 25 °C) 0,3 dl/g. Masse Molaire par GPC : 20 000 g/mol.
10 g de polymère sont ensuite mis à réagir à 0 °C dans 200 ml TFA avec 6 ml d'acide trifluorométhanesulfonique pendant 15 min. Lors de l'hydrolyse des groupements benzyliques, le polymère précipite sous forme de particules colloïdales. Après évaporation à sec, les particules sont reprises dans de l'eau et neutralisées à pH 7,4 avec de la soude. Une étape de dialyse assure l'élimination des sels du trifluoroacétate. Les particules sont enfin isolées par lyophilisation.
Rendement quantitatif. Analyse élémentaire [Na] 8,2 % (composition calculée 53 % GluONa).

### EXEMPLE 2 - PREPARATION DU POLYMERE POLY(CLUTAMATE DE SODIUM)-BLOC-(GLUTAMATE DE D'ETHYLE).

Ce polymère est préparé selon la méthode décrite dans l'exemple 1, utilisant les NCA du glutamate d'éthyle et du benzyle dans un rapport molaire 1:3. Après les étapes de polymérisation et de débenzylation, le polymère est purifié par dialyse à pH 7 et ensuite il est lyophilisé afin d'obtenir une poudre blanche.

### EXEMPLE 3 - PREPARATION DU POLYMERE POLY(GLUTAMATE DE SODIUM)-BLOC-(GLUTAMATE DE HEXADECYLE).

Ce polymère est préparé selon la méthode décrite dans l'exemple 1, utilisant les NCA du glutamate de dodecyle et du benzyle dans un rapport molaire 1:3. Après les étapes de polymérisation et de débenzylation, le polymère est purifié par dialyse à pH 7 et ensuite il est lyophilisé afin d'obtenir une poudre blanche.

### EXEMPLE 4 - PREPARATION DU POLYMERE POLY(GLUTAMATE DE SODIUM)-CO-(GLUTAMATE DE DODECYLE).

10 g d'acide polyglutamique (degré de polymérisation 120) sont dissous dans 200 ml de diméthylsulfoxide. On ajoute ensuite 15,5 g de KHCO₃ puis 2,87 ml de iodododécane. Le milieu réactionnel est maintenu à 60 °C pendant 40 heures sous flux d'azote. Le polymère est isolé par précipitation dans 1,5 l d'acide chlorhydrique 0,1 N, et le précipité ainsi formé lavé plusieurs fois dans de l'eau. La suspension colloïdale recherchée est obtenue en ajustant le pH du milieu à 7,4 par de la soude, puis déshydratée par lyophilisation. Le polymère lyophilisé est enfin lavé plusieurs fois dans de l'acétate d'éthyle, et séché sous vide à 50 °C. Composition par RMN (TFA-d) : 12% des acides glutamiques estérifiés par le dodécyle. Eau Résiduelle après lyophilisation 10.5%. Analyse élémentaire % (calc.) : C 41,29 (42, 15); H 5,99 (6,01) ; N 7,45 (7,63) ; Na 10,44 (10,45).

### EXEMPLE 5 - MISE EN EVIDENCE DES NANOPARTICULES PAR DIFFUSION DE LA LUMIERE (DDL) ET PAR MICROSCOPIE ELECTRONIQUE A TRANSMISSION (MET).

10 mg de particules du polymère 1 sont suspendus dans 10 ml d'eau ou une solution aqueuse de sel. Cette solution est ensuite introduite dans un granulomètre Coulter (ou diffractomètre laser). Les résultats de l'analyse de la granulométrie des différents produits testés sont présentés dans le tableau 1 suivant.

**Tableau 1 -**

| **Mesures de la taille des PV** | | |
|---|---|---|
| **EXEMPLE** | **POLYMERE** | **TAILLE (nm)** |
| Témoin | POLY[(GLU-O-NA)_{1,0}]_{X} | PAS DE PARTICULES (produit soluble) |
| **1** | POLY[(GLU-O-NA)_{0,63}-BLOC-(GLU-O-METHYL)_{0,37}]_{X} | 100 |
| **2** | POLY[GLU-O-NA)_{0,66}-BLOC-(GLU-O-ETHYL)_{0,34}]_{X} | 90 |
| **3** | POLY(GLU-O-NA)_{0,65}-BLOC-(GLU-O-HEXADECYLE)_{0,35}]_{X} | 110 |
| 4 | POLY[(GLU-O-NA)_{0,88}-CO-(GLU-O-DODECYLE)_{0,12}]_{X} | 15 |

### EXEMPLE 6 - TEST D'ASSOCIATION DES NANOPARTICULES AVEC UNE PROTEINE (L'INSULINE).

A partir d'une solution tampon phosphate isotonique de pH 7,4, on prépare une solution d'insuline humaine titrée à 1,4 mg/ml correspondant à 40 UI/ml. Dans 1 ml de cette solution d'insuline, on disperse 10 mg du **PV** préparé dans l'exemple 1. Après 15 heures d'incubation à température ambiante, l'insuline associée aux **PV** et l'insuline libre sont séparées par centrifugation (60 000 g, 1heure) et ultrafiltration (seuil de filtration 300 000 D). L'insuline libre récupérée dans le filtrat est dosée par CLHP ou par ELISA et l'on en déduit par différence la quantité d'insuline associée. La quantité d'insuline associée au **PV** est supérieure à 0,77 mg, ce qui représente plus de 55 % du total de l'insuline engagée.
Le tableau suivant rassemble les résultats des mesures de taux d'association effectuées sur différents **PV**. Le taux d'association exprime le pourcentage d'insuline associée par rapport à l'insuline engagée dans une préparation titrée à 1,4mg/ml d'insuline et 10 mg/ml de **PV**.

**Tableau 2 -**

| **Mesures du taux d'association avec l'insuline pour un mélange 0,14 mg insuline/mg PV** | | |
|---|---|---|
| **EXEMPLE** | **POLYMERE** | **TAUX D'ASSOCIATION (%)** |
| Témoins | POLY[(GLU-O-NA)_{1,0}]_{X} | 0 |
| **1** | POLY[(GLU-O-NA)_{0,63}-BLOC-(GLU-O-METHYL)_{0,37}]_{X} | 55 |
| **2** | POLY[GLU-O-NA)_{0,66}-DLOC-(GLU-O-ETHYL)_{0,34}]_{X} | 26 |
| **3** | POLY(GLU-O-NA)_{0,65}-BLOC-(GLU-O-HEXADECYLE)_{0,35}]_{X} | 36 |
| **4** | POLY[(GLU-O-NA)_{0,88}-CO-(GLU-O-DEODECYL)_{0,12}]_{X} | > 90 |

A titre comparatif, l'insuline ne s'associe pas avec le polyglutamate de sodium. Ce teste préliminaire sert à la détermination du mélange optimal en **PV** et en insuline pour obtenir une formulation optimale en **PV** et en insuline avec un haut taux de chargement.

### EXEMPLE 7 - DISSOCIATION ET CARACTERISATION DE LA PROTEINE APRES SA FORMULATION AVEC DES PV (L'INSULINE).

Une formulation est préparée à partir de **PV** et d'insuline, les quantités de chaque étant déterminées d'après les mesures de taux d'association dans l'exemple 6.
A partir d'une solution tampon phosphate isotonique de pH 7,4, on prépare une solution d'insuline humaine titrée à 2,8 mg/ml correspondant à 80 UI/ml. Dans 1 ml de cette solution d'insuline, on disperse 56 mg du **PV** préparé dans l'exemple 1. Le pH et l'isotonicité sont ajustés, si nécessaire, afin d'obtenir une formulation à pH 7,4 et 280-300 mOs.
Cette préparation est diluée dans des volumes croissants d'une solution à 0,5 % d'albumine bovine, isotonique et tamponnée à pH 7,4 par un tampon phosphate 0,01 M. La fraction d'insuline libérée est dosée par CLHP ou ELISA. Voir figure ci-jointe. La fraction libérée augmente avec la dilution. La totalité de l'insuline est libérée à partir de la dilution 20. Les dosages par CLHP et ELISA montrent que >90% de l'insuline est associée dans cette formulation.

### EXEMPLE 8 - TEST IN-VIVO AVEC DES PV-CHARGES D'UNE PROTEINE THERAPEUTIQUE (L'INSULINE).

Une formulation est préparée à partir de **PV** et d'insuline, les quantités de chaque étant déterminées d'après les mesures de taux d'association dans l'exemple 7.
Un groupe de 4 chiens beagle (mâles et femelles) pesant entre 10 et 12 kg sont mis à jeûn 18 heures. Une préparation est formulée selon l'exemple 8 et se compose de 80 UI d'insuline et 56 mg de **PV** (préparés selon l'exemple 1) dans 1 ml de tampon PBS. Les chiens reçoivent ensuite une administration sous-cutanée de cette préparation d'insuline à raison de 1 UI/kg de poids. Le sang est prélevé pour un dosage du glucose et d'insuline avant (-2 h, -1 h et 0 h) et après (1 h, 2 h, 4 h, 6 h, 12 h, 16 h, 20 h, 24 h, 28 h, 32 h, 36 h, 40 h, 44 h, 48 h) l'injection. Les concentrations en glucose sont mesurées dans les prélèvements par la méthode glucose-oxydase et l'insuline sérique est dosée en utilisant une méthode radio-immunologique.

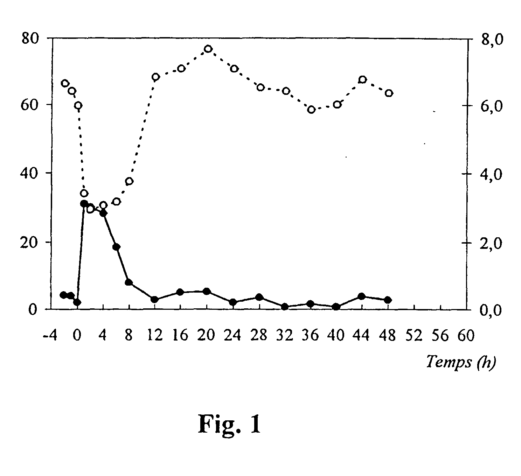

La **fig. 1** annexée trace la moyenne de l'évolution, en fonction du temps T en heures (h), du glucose et de l'insuline sérique pour les **PV** préparées selon l'exemple 1. La courbe -●- est celle de l'insulinémie exprimée en milli-unités internationales.
La courbe ---○--- est celle de la glycémie exprimée en mmol/l.
Le tableau 3 ci-après rassemble les résultats de la durée d'action d'insuline en présence des différents **PV** selon les exemples 1 à 5.

**Tableau 3 -**

| **Mesures du temps d'action de l'insuline (effet hypoglycémiant) en présence de PV selon l'invention** | | |
|---|---|---|
| EXEMPLE | POLYMERE | TEMPS DE RETOUR AU NIVEAU BASAL (h) |
| | INSULINE SOLUBLE (SANS PV) | 1 |
| | POLY[(GLU-O-NA)_{1,0}]_{X} | 1 |
| **1** | POLY[(GLU-O-NA)_{0,63}-BLOC-(GLU-O-METHYL)_{0,37}]_{X} | 12 |
| **2** | POLY[GLU-O-NA)_{0,66}-BLOC-(GLU-O-ETHYL)_{0,34}]_{X} | 15 |
| **3** | POLY(GLU-O-NA)_{0,65}-BLOC-(GLU-O-HEXADECYLE)_{0,35}]_{X} | 20 |
| **4** | POLY[(GLU-O-NA)_{0,88}-CO-(GLU-O-DODECYLE)_{0,12}]_{X} | 12 |

Cet exemple démontre la non dénaturation de l'insuline en présence de PV selon l'invention.
De plus, cet exemple 8 permet de mettre en évidence l'augmentation de la durée d'action de l'insuline, et donc, l'utilité des PV en tant que système retard pour la libération contrôlée de l'insuline. Elle montre également comment il est possible de maîtriser la durée d'action par la choix judicieuse du groupement hydrophobe.

## Revendications

1. Particules susceptibles d'être utilisées, notamment, pour la vectorisation (**PV**) de principes actifs (**PA**) du type de celles :
- à base de polyaminoacides (PAA) amphiphiles, linéaires, à enchaînements α-peptidiques ;
- aptes à se former spontanément par mise en contact des PAA avec un milieu liquide, de préférence avec de l'eau, dans lequel la partie hydrophile des PAA se solubilise plus que la partie hydrophobe desdits PAA, de manière que ces derniers précipitent en s'organisant en arrangements supra-moléculaires discrets ;
- de taille moyenne inférieure à 200 µm, de préférence à 100 µm et, plus préférentiellement encore, comprise entre 0,01 et 20 µm ;
- et aptes à s'associer avec au moins un **PA** et à relarguer celui-ci in vivo, de manière prolongée et contrôlée ;
**caractérisées :**
• **en ce que** les aminoacides récurrents (AAr) constitutifs de la chaîne principale des PAA sont identiques ou différents entre eux et sont choisis parmi les aminoacides naturels polaires que sont l'acide glutamique et l'acide aspartique et/ou parmi leurs sels, respectivement les glutamates et les aspartates,
• et **en ce que** certains de ces AAr sont porteurs chacun d'au moins un groupement R⁰ hydrophobe, ces groupements hydrophobes R⁰ étant identiques ou différents entre eux.

2. Particules selon la revendication 1, **caractérisées en ce que** R⁰ est distribué sur les chaînes de polymère de manière statistique ou ordonnée.

3. Particules selon la revendication 1 ou 2, **caractérisées en ce que** les groupements R⁰ liés aux AAr sont identiques ou différents entre eux et sont sélectionnés dans le groupe comprenant :
**(i)** les alkyles, les acyles ou les alcényles linéaires ou ramifiés, de préférence linéaires en C₁-C₂₀ et, plus préférentiellement encore, en C₂-C₁₈ ;
**(ii)** les groupements hydrocarbonés contenant un ou plusieurs hétéroatomes, de préférence ceux contenant de l'oxygène et/ou du soufre et, plus préférentiellement encore, ceux de formule suivante : 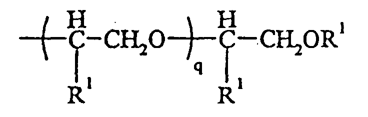 dans laquelle :
- les radicaux R¹ sont identiques ou différents entre eux et correspondent à l'hydrogène ou à un radical répondant à la même définition que celle donnée supra au point (i),
- q = 1 à 100 ;
**(iii)** les aryles, les aralkyles ou les alkylaryles, de préférence les aryles ;
**(iv)** les dérivés naturels hydrophobes, de préférence le cholestérol, les phosphatidylcholines et les diacylglycérols.

4. Particules selon la revendication 3, **caractérisées en ce que** les groupements R⁰ sont sélectionnés dans le groupe de radicaux suivants : méthyle, éthyle, propyle, hexyle, octyle, dodécyle, hexadécyle ou octadécyle.

5. Particules selon l'une quelconque des revendications 1 à 4, **caractérisées en ce que** chaque groupement hydrophobe R⁰ est relié à la chaîne d'AAr par une liaison covalente clivable, de préférence par hydrolyse .chimique et/ou enzymatique, ladite liaison étant plus spécialement de type ester et/ou amide.

6. Particules selon l'une quelconque des revendications 1 à 5, **caractérisées en ce que** la fraction des AAr₀ hydrophobes représente une proportion supérieure ou égale à 3 %, de préférence comprise entre 3 et 70 % et, plus préférentiellement encore, entre 3 et 60 %.

7. Particules selon l'une quelconque des revendications 1 à 6, **caractérisées en ce que** les PAA sont des polymères contenant, jusqu'à environ 1 000 AAr et, de préférence, jusqu'à environ 500 AAr.

8. Particules selon l'une quelconque des revendications 1 à 7, **caractérisées en ce que** ce sont des particules de vectorisation **PV** de taille moyenne comprise entre 0,01 et 0,5 µm, de préférence entre 0,01 et 0,2 µm.

9. Particules **caractérisées en ce qu'**elles sont obtenues par agrégation de particules selon l'une quelconque des revendications 1 à 8.

10. Particules selon l'une quelconque des revendications 1 à 8, **caractérisées en ce qu'**elles comprennent au moins un principe actif

11. Précurseurs des **PV** selon l'une quelconque des revendications 1 à 10, **caractérisés en ce qu'**il sont constitués par les PAA tels que définis dans l'une quelconque des revendications 1 à 7.

12. Suspension colloïdale, de préférence aqueuse, de particules selon l'une quelconque des revendications 1 à 10.

13. Procédé de préparation de la suspension colloïdale selon la revendication 12,
**caractérisé en ce qu'**il consiste, essentiellement :
**I** - à mettre en oeuvre des PAA amphiphiles linéaires et à enchaînements α-peptidiques d'aminoacides récurrents (AAr) sélectionnés parmi les aminoacides naturels polaires que sont l'acide glutamique et l'acide aspartique et/ou parmi leurs sels, respectivement les glutamates et les aspartates ; certains de ces AAr étant porteurs chacun d'au moins un groupement hydrophobe R⁰, ces groupements R⁰ étant identiques ou différents entre eux ;
**II** - à placer les PAA amphiphiles dans un milieu solvant, de préférence aqueux,
**III** - puis à rendre insolubles au moins les AAr porteurs de R⁰ dans le milieu, de manière à faire précipiter ces AAr et à former ainsi des arrangements supra moléculaire discrets.

14. Procédé selon la revendication 13, **caractérisé en ce que** l'on incorpore au moins un principe actif dans le milieu liquide contenant les particules **PV**, de manière à obtenir une suspension colloïdale de **PV** chargées en principe(s) actif(s).

15. Procédé selon la revendication 13 ou 14, **caractérisé en ce qu'**il comprend au moins une étape supplémentaire d'agrégation des particules, à l'aide d'au moins un agent d'agrégation constitué, par un sel et/ou un acide et/ou une base et/ou un polymère éventuellement ionique.

16. Précurseurs selon la revendication 11, particules selon l'une quelconque des revendications 1 à 10 ou suspension selon la revendication 12, comprenant au moins un principe actif médicamenteux qui est, de préférence, choisi parmi :
• les protéines et/ou les peptides parmi lesquels les plus préférentiellement retenus sont : les hémoglobines, les cytochromes, les albumines, les interférons, les antigènes, les anticorps, l'érythropoïétine, l'insuline, les hormones de croissance, le facteur IX, les interleukines ou leurs mélanges,
• les polysaccharides, l'héparine étant plus particulièrement sélectionnée,
• les acides nucléiques et, préférablement, les oligonucléotides d'ARN et/ou d'ADN,
• et leurs mélanges.

17. Précurseurs selon la revendication 11, particules selon l'une quelconque des revendications 1 à 10 ou suspension selon la revendication 12 contenant de l'insuline à titre de principe actif.

18. Précurseurs selon la revendication 11, particules selon l'une quelconque des revendications 1 à 10 ou suspension selon la revendication 12 contenant un principe actif constitué par au moins un vaccin.

19. Précurseurs selon la revendication 11, particules selon l'une quelconque des revendications 1 à 10 ou suspension selon la revendications 12 contenant au moins un principe actif formé par au moins un produit nutritionnel phytosanitaire ou cosmétique.

20. Spécialité pharmaceutique, nutritionnelle, phytosanitaire ou cosmétique, **caractérisée en ce qu'**elle comporte des précurseurs selon la revendication 11 et/ou des particules selon l'une quelconque des revendications 1 à 10, et/ou une suspension selon la revendication 12.

## Patentansprüche

1. Partikel die dazu geeignet sind, insbesondere für die Vektorisierung (PV) aktiver Bestandteile (PA) verwendet zu werden, wobei:
- die Partikel auf Basis linearer amphiphiler α-peptidisch verknüpfter Polyaminosäuren (PAA) sind;
- die Partikel dazu in der Lage, sich spontan zu bilden, wenn PAA mit einem flüssigen Medium, vorzugsweise mit Wasser, in Kontakt gebracht werden, in dem der hydrophile Teil der PAA sich besser löst als der hydrophobe Teil dieser PAA, sodass diese letzteren ausfallen, wobei sie sich in einzelnen supra-molekularen Anordnungen organisieren;
- die Partikel eine mittlere Größe kleiner als 200 µm, bevorzugt kleiner als 100 µm und noch weiter bevorzugt zwischen 0,01 und 20 µm aufweisen;
- und die Partikel dazu in der Lage sind, sich mit wenigstens einem PA zu assoziieren und diesen in vivo auf andauernde und kontrollierte Art und Weise freizusetzen;
**dadurch gekennzeichnet,**
- **dass** die wiederkehrenden Aminosäuren (AAr) welche die PAA-Hauptkette bilden gleich oder unterschiedlich voneinander sind und dass sie ausgewählt sind aus natürlichen polaren Aminosäuren wie Glutaminsäure und Asparaginsäure und/oder deren Salzen, bzw. den Glutamaten und den Aspartaten,
- und **dass** manche der AAr jeweils wenigstens eine hydrophobe Gruppierung R⁰ tragen, wobei diese hydrophoben Gruppierungen R⁰ gleich oder unterschiedlich voneinander sind.

2. Partikel nach Anspruch 1, **dadurch gekennzeichnet, dass** R⁰ statistisch oder geordnet auf die Polymerketten verteilt ist.

3. Partikel nach Anspruch 1 oder 2, **dadurch gekennzeichnet, dass** die mit den AAr verbundenen Gruppierungen R⁰ gleich oder unterschiedlich voneinander sind, und dass sie ausgewählt sind aus der Gruppe umfassend:
(i) lineare oder verzweigte Alkyl-, Acyl- oder Alkenylreste, bevorzugt lineare C₁-C₂₀ und noch weiter bevorzugt C₂-C₁₈ Reste;
(ii) Kohlenwasserstoff-Gruppierungen die ein oder mehrere Heteroatome enthalten, bevorzugt solche, die Sauerstoff und/oder Schwefel enthalten und noch weiter bevorzugt solche, die die folgende Formel aufweisen: 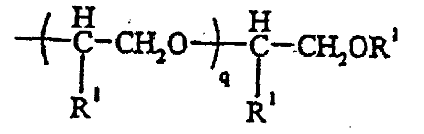 worin
- die Reste R¹ gleich oder unterschiedlich voneinander sind und Wasserstoff oder einen Rest mit der unter Punkt (i) angegebenen Definition bedeuten,
- q = 1 bis 100;
(iii) Aryl-, Aralkyl- oder Alkylaryl-, bevorzugt Arylreste;
(iv) natürliche hydrophobe Derivate, bevorzugt Cholesterin, Phosphatidylcholin und Diacylglycerin.

4. Partikel nach Anspruch 3, **dadurch gekennzeichnet, dass** die Gruppierungen R⁰ ausgewählt sind aus der Gruppe der folgenden Reste: Methyl, Ethyl, Propyl, Hexyl, Oktyl, Dodekyl, Hexadekyl oder Oktadekyl.

5. Partikel nach einem der Ansprüche 1 bis 4, **dadurch gekennzeichnet, dass** jede hydrophobe Gruppierung R⁰ durch eine spaltbare, bevorzugt durch chemische und/oder enzymatische Hydrolyse spaltbare, kovalente Bindung an die AAr-Kette gebunden ist, wobei die Bindung insbesondere eine Ester- und/oder Amid-Bindung ist.

6. Partikel nach einem der Ansprüche 1 bis 5, **dadurch gekennzeichnet, dass** der Anteil der hydrophoben AAr₀ größer oder gleich 3 % ist, bevorzugt zwischen 3 und 70 % und noch weiter bevorzugt zwischen 3 und 60 % liegt.

7. Partikel nach einem der Ansprüche 1 bis 6, **dadurch gekennzeichnet, dass** es sich bei den PAA um Polymere handelt, die bis zu etwa 1000 AAr und bevorzugt bis zu etwa 500 AAr aufweisen.

8. Partikel nach einem der Ansprüche 1 bis 7, **dadurch gekennzeichnet, dass** es sich um Vektorisierungspartikel PV mit einer mittleren Größe zwischen 0,01 und 0,5 µm, bevorzugt zwischen 0,01 und 0,2 µm handelt.

9. Partikel, **dadurch gekennzeichnet, dass** sie durch Aggregierung von Partikeln nach einem der Ansprüche 1 bis 8 erhalten sind.

10. Partikel nach einem der Ansprüche 1 bis 8, **dadurch gekennzeichnet, dass** sie wenigstens einen aktiven Bestandteil aufweisen.

11. Vorläufer von PV nach einem der Ansprüche 1 bis 10, **dadurch gekennzeichnet, dass** sie aus den wie in einem der Ansprüche 1 bis 7 definierten PAA gebildet sind.

12. Kolloidale bevorzugt wässrige Suspension von Partikeln nach einem der Ansprüche 1 bis 10.

13. Verfahren zur Herstellung der kolloidalen Suspension nach Anspruch 12,
**dadurch gekennzeichnet, dass** es im Wesentlichen besteht aus:
I. Umsetzen von amphiphilen linearen **PAA** die α-peptidisch verknüpfte wiederkehrende Aminosäuren (AAr) aufweisen die ausgewählt sind aus natürlichen polaren Aminosäuren wie Glutaminsäure und Asparaginsäure und/oder deren Salzen bzw. den Glutamaten und Aspartaten, wobei manche der AAr jeweils wenigstens eine hydrophobe Gruppierung R⁰ tragen, wobei diese Gruppierungen R⁰ gleich oder unterschiedlich voneinander sind;
II. Einbringen der amphiphilen PAA in ein bevorzugt wässriges Lösungsmedium,
III. anschließend wenigstens die AAr, welche R⁰ tragen, in dem Medium unlöslich machen, sodass diese AAr ausfallen und so einzelne supramolekulare Anordnungen bilden.

14. Verfahren nach Anspruch 13, **dadurch gekennzeichnet, dass** wenigstens ein aktiver Bestandteil in dem flüssigen Medium, das die PV-Partikel aufweist, enthalten ist, sodass eine kolloidale Suspension von PV, die mit einem oder mehreren aktiven Bestandteilen beladen sind, erhalten wird.

15. Verfahren nach einem der Ansprüche 13 oder 14, **dadurch gekennzeichnet, dass** es wenigstens einen zusätzlichen Schritt des Aggregierens von Partikeln mit Hilfe eines Aggregationsmittels umfasst, das aus einem Salz und/oder einer Säure und/oder einer Base und/oder einem gegebenenfalls ionischen Polymer gebildet ist.

16. Vorläufer nach Anspruch 11, Partikel nach einem der Ansprüche 1 bis 10 oder Suspension nach Anspruch 12, umfassend wenigstens einen aktiven medikamentösen Bestandteil, der bevorzugt ausgewählt ist aus:
- Proteinen und/oder Peptiden, wobei Hämoglobine, Cytochrome, Albumine, Interferone, Antigene, Antikörper, Erythropietin, Insulin, Wachstumshormone, Faktor IX, Interleukine oder deren Gemische am meisten bevorzugt sind,
- Polysacchariden, wobei insbesondere Heparin ausgewählt wird,
- Nukleinsäuren und vorzugsweise RNS- und/oder DNS-Oligonukleotiden,
- deren Gemischen.

17. Vorläufer nach Anspruch 11, Partikel nach einem der Ansprüche 1 bis 10 oder Suspension nach Anspruch 12, welche Insulin als den aktiven Bestandteil aufweisen.

18. Vorläufer nach Anspruch 11, Partikel nach einem der Ansprüche 1 bis 10 oder Suspension nach Anspruch 12, welche einen aus wenigstens einem Vakzin gebildeten aktiven Bestandteil aufweisen.

19. Vorläufer nach Anspruch 11, Partikel nach einem der Ansprüche 1 bis 10 oder Suspension nach Anspruch 12, welche wenigstens einen aktiven Bestandteil aufweisen der aus wenigstens einem Nahrungsmittel, Pflanzenschutzmittel oder Kosmetikprodukt gebildet ist.

20. Pharmazeutisches Mittel, Nahrungsmittel, Pflanzenschutzmittel oder Kosmetikprodukt **dadurch gekennzeichnet, dass** es Vorläufer nach Anspruch 11 und/oder Partikel nach einem der Ansprüche 1 bis 10 und/oder eine Suspension nach Anspruch 12 enthält.

## Claims

1. Particles which can be used in particular for the delivery (DPs) of active principles (APs), which are of the type of those:
- based on amphiphilic, linear polyamino acids (PAAs) with α-peptide chains;
- capable of forming spontaneously by bringing the PAAs into contact with a liquid medium, preferably with water, in which the hydrophilic portion of the PAAs solubilizes more than the hydrophobic portion of said PAAs, such that the latter precipitate while becoming organized in discrete supramolecular arrangements;
- having a mean size of less than 200 µm, preferably less than 100 µm, and even more preferably of between 0.01 and 20 µm;
- and capable of associating with at least one AP and of releasing the latter in vivo, in a sustained and controlled way;
**characterized:**
• **in that** the recurrent amino acids (rAAs) constituting the main chain of the PAAs are identical to or different from one another, and are chosen from natural polar amino acids, namely glutamic acid and aspartic acid, and/or from the salts thereof, respectively glutamates and aspartates,
• and **in that** some of these rAAs each bear at least one hydrophobic R⁰ group, these hydrophobic R⁰ groups being identical to or different from one another.

2. Particles according to Claim 1, **characterized in that** R⁰ is distributed on the polymer chains in a random or ordered manner.

3. Particles according to Claim 1 or 2, **characterized in that** the R⁰ groups attached to the rAAs are identical to or different from one another, and are selected from the group comprising:
(i) linear or branched, preferably linear, C₁-C₂₀, and even more preferably C₂-C₁₈, alkyls, acyls or alkenyls;
(ii) hydrocarbon-based groups containing one or more heteroatoms, preferably those containing oxygen and/or sulphur, and even more preferably those of the following formula: 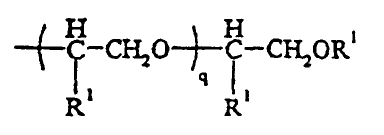 in which:
- the R¹ radicals are identical to or different from one another, and correspond to hydrogen or to a radical satisfying the same definition as that given above in point (i),
- q = 1 to 100;
(iii) aryls, aralkyls or alkylaryls, preferably aryls;
(iv) natural hydrophobic derivatives, preferably cholesterol, phosphatidylcholines and diacylglycerols.

4. Particles according to Claim 3, **characterized in that** the R⁰ groups are selected from the following group of radicals: methyl, ethyl, propyl, hexyl, octyl, dodecyl, hexadecyl or octadecyl.

5. Particles according to any one of Claims 1 to 4, **characterized in that** each hydrophobic R⁰ group is linked to the rAA chain via a covalent attachment which can be cleaved, preferably by chemical and/or enzymatic hydrolysis, said attachment being more especially of the ester and/or amide type.

6. Particles according to any one of Claims 1 to 5, **characterized in that** the fraction of hydrophobic rAA₀s represents a proportion greater than or equal to 3%, preferably between 3 and 70%, and even more preferably between 3 and 60%.

7. Particles according to any one of Claims 1 to 6, **characterized in that** the PAAs are polymers containing up to approximately 1000 rAAs, and preferably up to approximately 500 rAAs.

8. Particles according to any one of Claims 1 to 7, **characterized in that** they are delivery particles DP having a mean size of between 0.01 and 0.5 µm, preferably between 0.01 and 0.2 µm.

9. Particles **characterized in that** they are obtained by aggregation of particles according to any one of Claims 1 to 8.

10. Particles according to any one of Claims 1 to 8, **characterized in that** they comprise at least one active principle.

11. DP precursors according to any one of Claims 1 to 10, **characterized in that** they consist of the PAAs as defined in any one of Claims 1 to 7.

12. Colloidal, preferably aqueous, suspension of particles according to any one of Claims 1 to 10.

13. Process for preparing the colloidal suspension according to Claim 12,
**characterized in that** it consists, essentially:
I- in using linear amphiphilic PAAs containing α-peptide chains of recurrent amino acids (rAAs) selected from natural polar amino acids, namely glutamic acid and aspartic acid, and/or from the salts thereof, respectively glutamates and aspartates; some of these rAAs each bearing at least one hydrophobic R⁰ group, these R⁰ groups being identical to or different from one another;
II- in placing the amphiphilic PAAs in a solvent, preferably aqueous, medium;
III- and then in making at least the rAAs carrying R⁰ insoluble in the medium, so as to precipitate these rAAs and thus to form discrete supramolecular arrangements.

14. Process according to Claim 13, **characterized in that** at least one active principle is incorporated into the liquid medium containing the particles DP, so as to obtain a colloidal suspension of DPs loaded with active principle(s).

15. Process according to Claim 13 or 14, **characterized in that** it comprises at least one additional step of aggregation of the particles, using at least one aggregation agent consisting of a salt and/or an acid and/or a base and/or a polymer, optionally an ionic polymer.

16. Precursors according to Claim 11, particles according to any one of Claims 1 to 10 or suspension according to Claim 12, comprising at least one medicinal active principle which is preferably chosen from:
• proteins and/or peptides, among which the most preferably selected are: haemoglobins, cytochromes, albumins, interferons, antigens, antibodies, erythropoietin, insulin, growth hormones, factor IX, interleukins, or mixtures thereof,
• polysaccharides, heparin being more particularly selected,
• nucleic acids, and preferably oligonucleotides of RNA and/or of DNA,
• and mixtures thereof.

17. Precursors according to Claim 11, particles according to any one of Claims 1 to 10 or suspension according to Claim 12, containing insulin as an active principle.

18. Precursors according to Claim 11, particles according to any one of Claims 1 to 10 or suspension according to Claim 12, containing an active principle consisting of at least one vaccine.

19. Precursors according to Claim 11, particles according to any one of Claims 1 to 10 or suspension according to Claim 12, containing at least one active principle formed by at least one nutritional, plant-protection or cosmetic product.

20. Pharmaceutical, nutritional, plant-protection or cosmetic specialty, **characterized in that** it comprises precursors according to Claim 11 and/or particles according to any one of Claims 1 to 10 and/or a suspension according to Claim 12.
